# NEUE EUROPÄISCHE PATENTSCHRIFT

(11) **EP 3 509 493 B2**
(45) Veröffentlichungstag und Bekanntmachung des Hinweises auf die Entscheidung über den Einspruch: **10.01.2024**
(45) Hinweis auf die Patenterteilung: 17.02.2021
(21) Anmeldenummer: 17776950.2
(22) Anmeldetag: 12.09.2017
(51) Int. Cl.: A61B 6/00, G03C 5/17, G21K 4/00, H04N 5/32, G01T 1/20

(54) **SYSTEM UND VERFAHREN ZUR BEREITSTELLUNG VON AUFNAHMEPARAMETERN**
SYSTEM AND METHOD FOR PROVIDING IMAGING PARAMETERS
SYSTÈME ET PROCÉDÉ POUR L'OBTENTION DE PARAMÈTRES DE PRISE DE VUE

(30) Priorität: 12.09.2016 DE 102016117051
(43) Veröffentlichungstag der Anmeldung: 17.07.2019
(60) Teilanmeldung: 21157489.2
(73) Patentinhaber: Dürr Dental SE, 74321 Bietigheim-Bissingen (DE)
(72) Erfinder: WEBER, Michael, 71576 Burgstetten (DE); PHILIPPS, Bernd, 74199 Untergruppenbach (DE)
(74) Vertreter: Ostertag & Partner Patentanwälte mbB
(86) Internationale Anmeldenummer: PCT/EP2017/072839
(87) Internationale Veröffentlichungsnummer: WO 2018/046749

(56) Entgegenhaltungen:
- EP-A1- 0 908 762
- EP-A1- 2 386 904
- WO-A1-2010/109064
- US-A- 5 264 684
- US-A- 5 757 021
- US-A1- 2012 019 369
- US-B2- 7 355 195
- Anonymous: "DIRECTIVES COUNCIL DIRECTIVE 2013/59/EURATOM of 5 December 2013", Official Journal of the European Union, 17 January 2014 (2014-01-17), pages 1-73,

## Beschreibung

Die Erfindung betrifft ein System mit einer Röntgenaufnahmevorrichtung zur Aufnahme eines Röntgenbildes auf einer Speicherfolie und einer Auslesevorrichtung für die Speicherfolie. Solche Systeme werden heutzutage in der Röntgentechnik, beispielsweise in der Dentalmedizin, zur Aufnahme von Röntgenbildern verwendet. Die Speicherfolie weist zur Speicherung des Röntgenbildes ein Phosphormaterial auf, das in einer transparenten Matrix eingebettet ist. Dadurch entstehen Speicherzentren, die durch einfallendes Röntgenlicht in angeregte metastabile Zustände gebracht werden können. Belichtet man eine solche Speicherfolie in einer Röntgenapparatur, beispielsweise zur Aufnahme eines Bissflügels eines Patienten, so enthält die Speicherfolie ein latentes Röntgenbild in Form angeregter und nicht angeregter Speicherzentren.

Zum Auslesen der Speicherfolie wird diese in einer Auslesevorrichtung, beispielsweise einer Scanvorrichtung, punktweise mit Ausleselicht abgetastet, wodurch die metastabilen Zustände der angeregten Speicherzentren in einen Zustand gebracht werden, der unter Abgabe von Fluoreszenzlicht relaxiert. Dieses Fluoreszenzlicht wird mithilfe einer Detektoreinheit erfasst, sodass mit einer entsprechenden Auswerteelektronik das Röntgenbild sichtbar wird.

In der Medizin ist eine eindeutige Nachverfolgung und Zuordnung der Speicherfolien extrem wichtig. Zu diesem Zweck werden hier - wie auch in vielen anderen Logistikbereichen - Identifizierungssysteme eingesetzt.

US 2012/0019369 A1 beschreibt ein medizinisches Bildgebungssystem, welches zumindest eine Röntgenspeicherfolie, eine Röntgenvorrichtung zum Erstellen eines Bildes eines Objekts auf dieser Röntgenspeicherfolie und eine Auslesevorrichtung zum Auslesen von Bildinformationen, die auf der Röntgenspeicherfolie gespeichert sind.

Aus EP 0 908 762 A1 geht ein kombiniertes System zum Identifizieren eines Röntgenbildes, wobei das Röntgenbild in einem photostimulierbaren Phosphorschirm gespeichert wurde, und zum Anzeigen eines Vorschaubildes des Röntgenbildes auf einem einzelnen Computer hervor.

Ein Röntgenspeicherfolien-Patienteninformationssystem, das eine portable Röntgeneinheit umfasst, ist in US 5,264,684 beschrieben.

Es ist eine Aufgabe der Erfindung, ein System und ein Verfahren zur Bereitstellung von Informationen für eine Auslesevorrichtung anzugeben, das einfach zu handhaben, zuverlässig und kostengünstig ist.

Des Weiteren ist es von Interesse, den Auslesevorgang der Speicherfolie effizient und genau zu gestalten.

Diese Aufgabe wird durch ein System gemäß dem unabhängigen Anspruch 1, sowie durch ein Verfahren gemäß dem unabhängigen Verfahrensanspruch gelöst. Weitere Ausgestaltungen der Erfindung sind in den abhängigen Ansprüchen angegeben.

Das erfindungsgemäße System weist eine Röntgenaufnahmevorrichtung zur Aufnahme eines Röntgenbildes auf einer Speicherfolie und eine Auslesevorrichtung für die Speicherfolie auf. Es ist vorgesehen, dass die Speicherfolie einen Datenträger aufweist. Des Weiteren ist vorgesehen, dass die Röntgenaufnahmevorrichtung oder/und die Auslesevorrichtung eine Datenvorrichtung mit einer Schreib-/Lesevorrichtung zum Schreiben von die Röntgenaufnahme betreffenden Aufnahmeparametern auf den Datenträger und zum Lesen von auf dem Datenträger gespeicherten Informationen umfasst, wobei die Schreib-/Lesevorrichtung dazu eingerichtet ist, die gelesenen Informationen an die Auslesevorrichtung zu übertragen, so dass der Auslesevorrichtung die bei der Aufnahme des Röntgenbildes verwendeten Aufnahmeparameter für ein Auslesen der Speicherfolie vor dem Auslesevorgang zur Verfügung stehen. Die Aufnahmeparameter umfassen eine Spannung, eine Stromstärke, eine Belichtungszeit, eine Dosis, ein Dosisflächenprodukt und/oder einen Blendenwert. Ferner kann das Auslesen der Speicherfolie auf die Aufnahmeparameter angepasst werden.

Es ist somit mit dem erfindungsgemäßen System möglich, für eine Aufnahme eines Röntgenbilds mit der Speicherfolie entsprechende Informationen dem Datenträger zu entnehmen. Dabei kann es sich beispielsweise um Aufnahmeparameter handeln, die für die Aufnahme mit dem Röntgengerät zu verwenden sind. Des Weiteren kann mittels der auszulesenden Informationen die Verwendungsanzahl der Speicherfolie erfasst werden, um einen mutmaßlichen Verschleiß der Speicherfolie berechnen oder abschätzen zu können. Es stehen der Auslesevorrichtung für die Speicherfolie beispielsweise bereits vor dem Auslesevorgang die auf dem Datenträger befindlichen Informationen zur Verfügung und das Auslesen der Speicherfolie kann beispielsweise bereits auf die Aufnahmeparameter angepasst werden, die bei der Aufnahme des Röntgenbildes verwendet wurden. Ein besonderer Vorteil ergibt sich daraus, dass das Schreiben der Aufnahmeparameter durch eine Datenvorrichtung der Röntgenaufnahmevorrichtung durchführbar ist. Dies ermöglicht eine weitgehende Automation des Schreibvorgangs auf die Speicherfolie mit den entsprechenden Vorteilen wie eine geringe Fehlerrate und eine weitgehende Geheimhaltung möglicherweise sensibler Daten.

Bei einer Ausführungsform des Systems kann vorgesehen sein, dass die Informationen einen die Speicherfolie eindeutig identifizierenden Identifikationscode darstellen. Somit kann durch ein Auslesen der auf den Datenträger gespeicherten Informationen die Speicherfolie eindeutig identifiziert werden. Dies ermöglicht eine speicherfolienbezogene Verknüpfung anderweitig erfasster Daten wie beispielsweise die Generierung und/oder Erfassung von Verschleißdaten. Des Weiteren können mit der eindeutigen Identifizierung auch beispielsweise damit verknüpfte und anderweitig gespeicherte Daten der Speicherfolie oder dem darauf gespeichertem Röntgenbild verknüpft werden. Es können auch zusätzlich auf dem Datenträger gespeicherte Informationen wie beispielsweise Aufnahmeparameter, Verschleißdaten oder ähnliches mit der eindeutigen Identifizierung verknüpft werden.

Es ist vorgesehen, dass die Datenvorrichtung eine Schreib-/Lesevorrichtung zum Schreiben von die Röntgenaufnahme betreffenden Aufnahmeparametern auf den Datenträger umfasst. Es ist mittels der Schreib-/Lesevorrichtung möglich, eine Aufnahme eines Röntgenbilds mit der Speicherfolie aufzunehmen und die dabei verwendeten Aufnahmeparameter auf den zur Speicherfolie zugehörigen Datenträger abzuspeichern. Gleichzeitig oder alternativ können die auf dem Datenträger befindlichen Aufnahmeparameter von der Schreib-/Lesevorrichtung ausgelesen werden. Es ist somit ein von einer zentralen Informationsverarbeitung unabhängiger Informationstransfer alleine über den mit der Speicherfolie verbundenen Datenträger möglich. Dies sichert zum einen die lückenlose Nachverfolgung der mit der Aufnahme des Röntgenbildes auf der Speicherfolie verbundenen Aufnahmeparameter. Zum anderen kann eine ständige zentrale Anbindung des Systems an eine Datenbank oder ähnliches entfallen.

Es kann außerdem vorgesehen sein, dass die Röntgenaufnahmevorrichtung dazu eingerichtet ist, die Aufnahmeparameter and die Schreib-/Lesevorrichtung zu übertragen. Es können somit die bei der Röntgenaufnahme verwendeten Aufnahmeparameter direkt auf den Datenträger gespeichert werden.

Bei den Aufnahmeparametern handelt es sich um eine Spannung, eine Stromstärke, eine Belichtungszeit, eine Dosis, ein Dosisflächenprodukt oder/und einen Blendenwert. Das Aufzeichnen der genannten Aufnahmeparameter stellt eine Verknüpfung zwischen den genannten Parametern und dem auf der Speicherfolie befindlichen Röntgenbild dar und erlaubt somit eine Nachverfolgung der Röntgenaufnahme, ohne dass eine zentralisierte Dateninfrastruktur vorgehalten werden müsste.

Bei einer besonders bevorzugten Ausführungsform ist vorgesehen, dass der Datenträger ein RFID-Transponder ist. Dementsprechend kann vorgesehen sein, dass es sich bei der Schreib-/Lesevorrichtung um ein RFID-Schreib-/Lesegerät handelt. Die RFID-basierte Verbindungstechnologie kann beispielsweise gemäß der Norm ISO/IEC 18000-x arbeiten, ist aber nicht auf diese Norm eingeschränkt.

Die erfindungsgemäße Speicherfolie ist für ein solches System ausgelegt und weist eine lichtempfindliche Schicht, insbesondere für die Speicherung eines Röntgenbildes auf und ist dazu ausgelegt, in einer Lichtschutzhülle aufbewahrt zu werden. Es ist vorgesehen, dass die Speicherfolie einen Datenträger aufweist, der dazu ausgelegt ist, Aufnahmeparameter einer Röntgenaufnahme zu speichern und für ein Auslesen bereit zu stellen. Somit ergeben sich die bereits oben erläuterten Vorteile.

Das erfindungsgemäße Verfahren zur Bereitstellung von Informationen für eine Auslesevorrichtung weist die folgenden Schritte auf: Es wird mittels einer Röntgenaufnahmevorrichtung ein Belichtungsvorgang einer Speicherfolie durchgeführt, sodass ein Röntgenbild auf der Speicherfolie entsteht. Es werden die bei der Röntgenaufnahme verwendeten Aufnahmeparameter auf dem der Speicherfolie fest zugeordneten Datenträger geschrieben, wobei die Aufnahmeparameter eine Spannung, eine Stromstärke, eine Belichtungszeit, eine Dosis, ein Dosisflächenprodukt und/oder einen Blendenwert umfassen. Es wird der Datenträger ausgelesen. Es wird die Speicherfolie unter Berücksichtigung des Ausleseergebnisses des Datenträgers ausgelesen. Es kann vor oder nach dem Belichten eine die Speicherfolie charakterisierende Kennzeichnung der Speicherfolie von dem Datenträger gelesen werden. Somit können die bei einer Röntgenaufnahme verwendeten Aufnahmeparameter auf dem Datenträger gespeichert und beispielsweise beim Auslesen der Speicherfolie oder bei einer Interpretation des auf der Speicherfolie befindlichen Röntgenbildes ausgelesen und verwendet werden, ohne dass ein zentralisierter Datenfluss stattfinden muss. Dabei umfasst der Schritt des Beschreibens des Datenträgers das Abspeichern von Informationen, welche den Belichtungsvorgang charakterisieren. Dabei handelt es sich um die Aufnahmeparameter bei der Röntgenaufnahme. Das Auslesen der Speicherfolie berücksichtigt das Ausleseergebnis des Datenträgers. Werden bei dem Auslesen der Speicherfolie beispielsweise die Aufnahmeparameter in Betracht gezogen, mit denen das Röntgenbild auf die Speicherfolie belichtet wurde, können unter Umständen die Auslesebedingungen für die Speicherfolie optimiert werden. Dabei ist es relevant, dass die Röntgenvorrichtung die Aufnahmeparameter mittels der Datenvorrichtung direkt in den Datenträger der Speicherfolie speichert. Somit sind keine manuellen Eingaben erforderlich und es ergibt sich ein hohes Maß an Sicherheit bezüglich der Richtigkeit der auf dem Datenträger gespeicherten Informationen. Es können also absichtliche oder unabsichtliche Falscheingaben vermieden werden. Gleichzeitig wird der gesamte Arbeitsablauf im Vergleich zu dem Fall, in dem beispielsweise manuelle Eingaben vorgenommen werden müssten, zeitlich verkürzt und hinsichtlich der Sicherheit verbessert. Die Vorteile einer dezentralen Speicherung bieten sich gleichzeitig an - schnelle Verfügbarkeit der Daten, keine aufwändige Anbindung an ein zentrales System, etc.

Die Erfindung wird nun unter Bezugnahme auf die beigefügten Zeichnungen näher erläutert. Es zeigen:
- Figuren 1A, 1B: in schematischer Darstellung Teile eines erfindungsgemäßen Systems;
- Figuren 2A-D: in schematischen Darstellung verschiedene Ausführungsformen einer erfindungsgemäßen Speicherfolie;
- Figur 3: in einer schematischen Darstellung eine Ausführungsform einer Auslesevorrichtung; und
- Figur 4: ein Ablaufdiagramm eines erfindungsgemäßen Verfahrens.

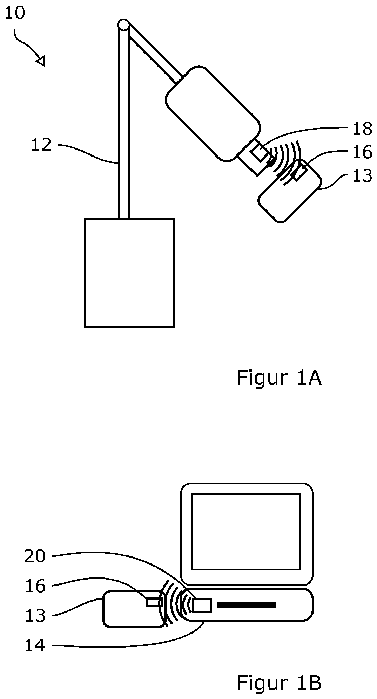

In den Figuren 1A und 1B ist ein System 10 zur Bereitstellung von Informationen dargestellt. Das System 10 umfasst eine Röntgenaufnahmevorrichtung 12 (Figur 1A) zur Belichtung einer Speicherfolie 13 mit einem Röntgenbild, wie sie beispielsweise in der Dentalmedizin eingesetzt werden, sowie eine Auslesevorrichtung 14 zum Auslesen des auf der Speicherfolie 13 befindlichen Röntgenbildes. Zur Aufnahme eines Röntgenbildes wird die Speicherfolie 13 üblicherweise in der Mundhöhle eines Patienten an geeigneter Stelle mittels nicht dargestellter Haltevorrichtungen angeordnet und über die Röntgenaufnahmevorrichtung 12 belichtet. Für die Belichtung sind an der Röntgenaufnahmevorrichtung 12 für die jeweilige Aufnahmesituation geeignete Aufnahmeparameter einzustellen. Diese Aufnahmeparameter umfassen beispielsweise eine Spannung, eine Stromstärke, eine Belichtungszeit, eine Dosis, ein Dosisflächenprodukt und/oder einen Blendenwert und bestimmen so die Aufnahmebedingungen. Unter den Aufnahmeparametern können sich aber auch patienten- oder auftragsspezifische Informationen befinden.

Die Speicherfolie 13 umfasst neben der eigentlichen röntgenstrahlenempfindlichen Struktur einen RFID-Transponder 16. Der RFID-Transponder 16 kann beispielsweise an oder in einer üblicherweise vorgesehenen lichtdichten Schutzhülle angeordnet sein. Der RFID-Transponder 16 arbeitet mit einer Schreib-/Lesevorrichtung 18 an der Röntgenaufnahmevorrichtung 12 und einem Lesegerät 20 an der Auslesevorrichtung 14 zusammen.

Die an der Röntgenaufnahmevorrichtung 12 vorgesehene Schreib-/Lesevorrichtung 18 ist dazu ausgelegt, einen Teil oder alle Aufnahmeparameter auf den RFID-Transponder 13 zu schreiben. Dazu können beispielsweise die vor dem Belichtungsvorgang eingestellten Sollwerte oder/und während oder nach dem Belichtungsvorgang erfasste Messwerte als Aufnahmeparameter erfasst und auf den RFID-Transponder 16 geschrieben werden. Zusätzlich kann die Schreib-/Lesevorrichtung 18 auch auf dem RFID-Transponder befindliche Informationen auslesen. Beispielsweise können bereits bei der Vorbereitung der Röntgenaufnahme auf der Speicherfolie 13 beispielsweise den Patienten, den Auftrag, das Röntgensystem oder/und das Gesamtsystem betreffende oder ähnliche Informationen auf dem RFID-Transponder 16 abgespeichert werden, die dann von der Röntgenaufnahmevorrichtung 12 ausgelesen und gegebenenfalls in der Konfigurierung des Belichtungsvorgangs der Speicherfolie 13 Eingang finden können.

Das Schreiben der Informationen durch die Röntgenaufnahmevorrichtung 12 mittels der Schreib-/Lesevorrichtung 18 auf den RFID-Transponder 13 kann auch das Schreiben der tatsächlich verwendeten Aufnahmeparameter oder/und das Schreiben von gemessenen Messwerten umfassen. Das Schreiben kann unabhängig von einem Bedienvorgang automatisch am Ende eines Aufnahmevorgangs erfolgen.

Nach der erfolgten Belichtung muss das auf der Speicherfolie 13 befindliche Röntgenbild ausgelesen werden. Hierfür ist in der gezeigten Ausführungsform des Systems 10 die Auslesevorrichtung 14 vorgesehen. Bei der Auslesevorrichtung 14 kann es sich beispielsweise um eine Scanvorrichtung handeln, die mittels eines geführten Laserstrahls die metastabilen Zustände in der Speicherfolienmatrix aktiviert und so ein Auslesen des Röntgenbildes ermöglicht. Die in dem RFID-Transponder 16 enthaltenen Aufnahmeparameter können mittels des an der Auslesevorrichtung 14 vorgesehenen Lesegeräts 20 beispielsweise bereits vor dem Auslesevorgang der Speicherfolie 13 ausgelesen werden und gegebenenfalls für den Auslese-/Scanvorgang verwendet werden. Die Kenntnis der Aufnahmeparameter kann die Einstellung des Auslesevorgangs unter Umständen erleichtern.

Das bei der Auslesevorrichtung 14 vorgesehene Lesegerät 20 kann auch als Schreib-/Lesevorrichtung ähnlich der Schreib-/Lesevorrichtung 18 ausgeführt sein. So können nach dem Auslesen der Speicherfolie 13 noch auf dem RFID-Transponder 16 befindliche Informationen wieder gelöscht werden. Alternativ oder zusätzlich können ein Teil oder die gesamten Ausleseergebnisse wiederum auf den RFID-Transponder 16 geschrieben werden und so abgespeichert werden. Es kann auch ein Vermerk auf dem RFID-Transponder 16 abgelegt werden, der darauf hinweist, dass die Speicherfolie 13 bereits ausgelesen wurde.

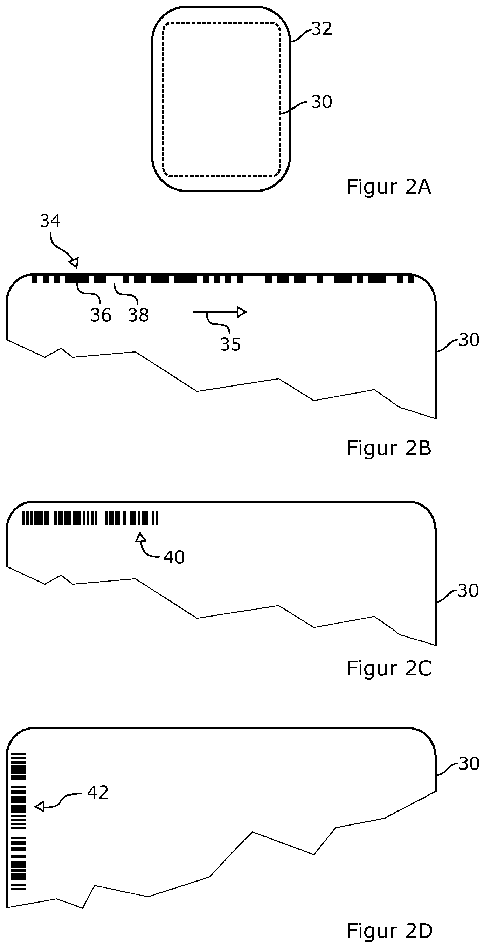

Die Figuren 2A-D zeigen eine Ausführungsform einer Speicherfolie 30. Die Speicherfolie 30 ist, wie in Figur 2A gezeigt, während ihrer Handhabung in einer Schutzhülle 32 eingeschoben. Die Schutzhülle 32 dient als mechanischer Schutz, um die empfindliche Speicherfolie 30 vor Verkratzen oder Knicken zu schützen. Gleichzeitig schützt die Schutzhülle 32 die Speicherfolie 30 vor unerwünschtem Lichteinfall, der das auf der Speicherfolie 30 befindliche latente Speicherbild zerstören oder eine noch unbelichtete Speicherfolie in unerwünschter Weise belichten würde. Für ein Auslesen ist die Speicherfolie 30 der Schutzhülle in geschützter Umgebung zu entnehmen und wird mit einem Ausleselicht punkt- oder zeilenweise abgetastet, wodurch die das Röntgenbild speichernden metastabilen Zustände der angeregten Speicherzentren relaxieren und Fluoreszenzlicht abgeben.

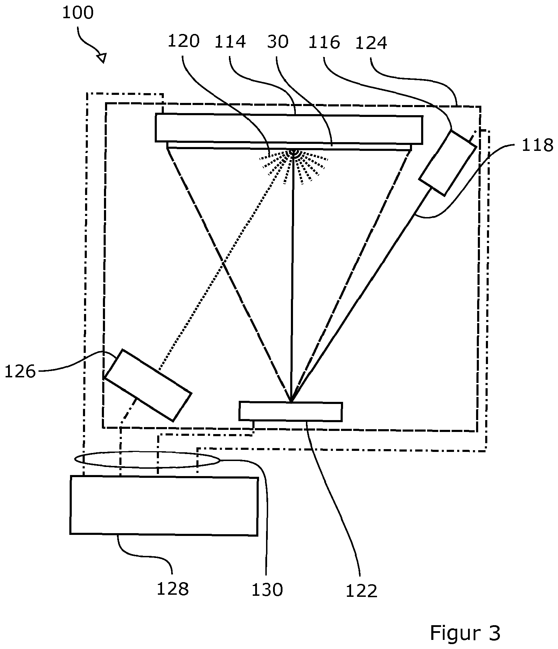

Figur 3 zeigt eine Scanvorrichtung 100 zum Auslesen einer solchen Speicherfolie 30, die in Form von durch Röntgenstrahlung angeregte metastabile Speicherzentren ein latentes Röntgenbild trägt.

Die Scanvorrichtung 100 weist eine Stützvorrichtung 114 für die Speicherfolie 30 auf. Beispielsweise kann die Speicherfolie 30 auf der Stützvorrichtung 114 mit Unterdruck so befestigt sein, dass die Speicherfolie 30, die im Allgemeinen flexibel ist, sich plan an die Stützfläche 114 anschmiegt.

Die Scanvorrichtung 100 umfasst ferner als Ausleselichtquelle einen Laser 116, der einen Ausleselichtstrahl 118 mit einer im Roten liegenden Wellenlänge erzeugt, mit dem die metastabilen Speicherzentren der Speicherfolie 30 zu Fluoreszenz angeregt werden können. Dieses Fluoreszenzlicht 120 liegt typischerweise im Blauen.

In der vorliegenden Ausführungsform der Scanvorrichtung 100 ist der Laser 116 so angeordnet, dass er den Ausleselichtstrahl 118 auf eine steuerbare Ablenkeinheit richtet. Die steuerbare Ablenkeinheit ist vorliegend als Spiegel 122 aus-gebildet. Es sind aber auch andere Ablenkeinheiten außer Spiegel wie etwa Optiken oder dergleichen denkbar. Der Spiegel 122 kann als Mikrospiegel, insbesondere als MEMS-Komponente ausgeführt sein und so ein Abtasten der Fläche der Speicherfolie 30 ohne oder mit nur geringer Relativbewegung zwischen Spiegel 122 und Stützvorrichtung 114 ermöglichen. Alternativ kann der Spiegel 122 auch herkömmlich als rotierender Spiegel für einen Trommelscanner vorgesehen sein. In diesem Fall ist eine Relativbewegung zwischen der Stützvorrichtung 114 und dem Spiegel 122 mittels einer Transportvorrichtung (nicht abgebildet) realisiert.

Die Scanvorrichtung 100 kann ferner einen in der Zeichnung gestrichelt angedeuteten Reflektor 124 umfassen, der den gesamten Messraum um die Speicherfolie 30 herum lichtdicht umschließt, sodass das von der Speicherfolie 30 ausgehende Fluoreszenzlicht 120 zu einem Photodetektor 126 reflektiert wird. Um zu verhindern, dass gestreutes Ausleselicht 118 in den Photodetektor 126 gelangt, können geeignete Maßnahmen wie etwa ein dichroitisches Filtermaterial vorgesehen sein.

Zur Steuerung des Auslesevorgangs umfasst die Scanvorrichtung 100 eine Steuereinheit 128, die beispielsweise neben der Steuerfunktion auch Auswerte- oder Korrekturfunktionen wahrnehmen kann. Die Steuereinheit 128 selbst oder die Auswerte- oder/und Korrekturfunktonen können aber auch auf einem separaten Computer implementiert sein. Die Steuereinheit 128 ist mittels Leitungen 130 mit der Stützvorrichtung 114, dem Detektor 126, dem Laser 116 sowie dem Spiegel 122 verbunden.

Zum Auslesen steuert die Steuereinheit 128 den Laser 116 sowie den Spiegel 122 an und tastet die Speicherfolie 30 punktweise sequenziell mit dem Ausleselichtstrahl 118 ab. Dabei wird die Intensität des abgegebenen Fluoreszenzlichts 120 mithilfe des Photodetektors 126 erfasst und in der Steuereinheit 128 zur Ausgabe aufbereitet.

In den Figuren 2B-D sind drei verschiedenen Ausführungsformen einer Speicherfolie 30 dargestellt. Die in Figur 2B dargestellte Speicherfolie 30 weist an ihrer Oberkante eine Strichcode-Struktur 34 auf, die im Wesentlichen die komplette Breite der Speicherfolie 30 in Richtung 35 einer Abtastzeile überdeckt. Die Strichcode-Struktur 34 ist so ausgebildet, dass sie bei einem Auslesen der Speicherfolie wie beispielsweise mit der Scanvorrichtung 100 in Abtastrichtung des Ausleselichtstrahls 118 Bereiche 36 mit erhöhter Reflektivität für den Ausleselichtstrahl 118 und Bereiche 38 mit geringerer Reflektivität, beispielweise mit normaler Reflektivität, aufweist. Die Bereiche 36 mit erhöhter Reflektivität können beispielsweise als Streubereiche wirken. Bei einem Abtasten der Speicherfolie kann somit beispielsweise vor einem normalen Auslesevorgang das von der Strichcode-Struktur 34 bei einem zeilenweisen Abtasten entstehende Streulicht detektiert werden. Da es bei diesem Vorgang nicht auf eine hohe Ortsauflösung ankommt, kann das Streulicht beispielsweise mit einer einfachen Photodiode (nicht abgebildet) erfasst werden. Alternativ oder zusätzlich kann eventuell bei der geforderten geringen Empfindlichkeit auch der ohnehin vorhandene Photodetektor 1126 diese Aufgabe übernehmen.

Die Figuren 2C und D zeigen Abwandlungen. Im Unterschied zur Strichcode-Struktur der Figur 2B bedeckt die Strichcode-Struktur 40 nur einen Teil der Oberfläche der Speicherfolie 30 in Abtastrichtung 35. Bei der in Figur 2D gezeigten Abwandlung erstreckt sich eine Strichcode-Struktur 42 senkrecht zur zeilenweisen Abtastrichtung 35 und erfordert somit ein Detektieren des entstehenden Streulichts zu Anfang jeder Abtastzeile.

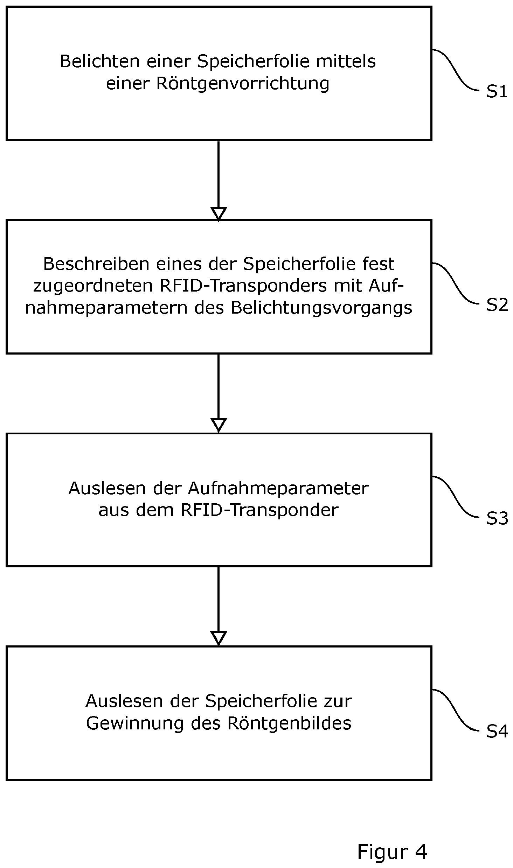

Figur 4 beschreibt eine Ausführungsform eines Verfahrens zur Bereitstellung von Informationen für eine Auslesevorrichtung. Das Verfahren weist folgende Schritte auf:
Eine Speicherfolie wird mittels einer Röntgenvorrichtung belichtet (S1). Bei dem Belichtungsvorgang wird ein Röntgenbild latent in der Speicherfolie erzeugt.

Ein der Speicherfolie fest zugeordneter RFID-Transponder wird mit Aufnahmeparametern des Belichtungsvorgangs beschrieben (S2). Der Vorgang des Beschreibens (S2) kann bereits vor dem Schritt des Belichtens (S1) stattfinden, wenn auf dem RFID-Transponder ausschließlich einzustellende Sollwerte zu hinterlegen sind. Alternativ oder zusätzlich kann der Vorgang des Beschreibens (S2) während oder nach dem Belichtungsvorgang (S1) stattfinden und es können alternativ oder zusätzlich auch während des Belichtungsvorgangs (S1) erfasste Messwerte auf den RFID-Transponder gespeichert werden. Für das Beschreiben des RFID-Transponders kann die Speicherfolie noch in der Röntgenvorrichtung verbleiben oder der Röntgenvorrichtung bereits entnommen sein.

Die auf dem RFID-Transponder befindlichen Aufnahmeparameter werden ausgelesen (S3). Die Speicherfolie kann nach dem Ende des Belichtungsvorgangs (S1) und dem Beschreiben des RFID-Transponders (S2) zu einer Auslesevorrichtung verbracht werden, um dort die Aufnahmeparameter auszulesen.

Das auf der Speicherfolie befindliche Röntgenbild wird mittels einer geeigneten Auslesevorrichtung ausgelesen (S4). Dabei kann es sich beispielsweise um eine Scanvorrichtung handeln, die mittels eines Lasers das latente Röntgenbild aktiviert und so ein Auslesen ermöglicht. Die Schritte des Auslesens der Aufnahmeparameter (S3) und des Auslesens der Speicherfolie (S4) können unabhängig voneinander erfolgen. Es werden vor dem Auslesen der Speicherfolie (S4) die Aufnahmeparameter aus dem RFID-Transponder ausgelesen (S3), um aus den Aufnahmeparametern Rückschlüsse auf geeignete Einstellungen für das Auslesen der Speicherfolie zu gewinnen.

## Patentansprüche

1. System (10) mit
- einer Röntgenaufnahmevorrichtung (12) zur Aufnahme eines Röntgenbildes auf einer Speicherfolie (13) und
- einer Auslesevorrichtung (14) für die Speicherfolie (13), wobei die Speicherfolie (13) einen Datenträger (16) aufweist, wobei
die Röntgenaufnahmevorrichtung (12) oder/und die Auslesevorrichtung (14) eine Datenvorrichtung (18, 20) mit einer Schreib-/Lesevorrichtung zum Schreiben von die Röntgenaufnahme betreffenden Aufnahmeparametern auf den Datenträger (16) und zum Lesen von auf dem Datenträger (16) gespeicherten Informationen umfasst, wobei die Aufnahmeparameter eine Spannung, eine Stromstärke, eine Belichtungszeit, eine Dosis, ein Dosisflächenprodukt und/oder einen Blendenwert umfassen, wobei
die Schreib-/Lesevorrichtung (20) dazu eingerichtet ist, die gelesenen Informationen an die Auslesevorrichtung (14) zu übertragen, so dass der Auslesevorrichtung (14) die bei der Aufnahme des Röntgenbildes verwendeten Aufnahmeparameter für ein Auslesen der Speicherfolie (13) vor dem Auslesevorgang zur Verfügung stehen, und wobei
das Auslesen der Speicherfolie (13) an die Aufnahmeparameter angepasst werden kann.

2. System nach Anspruch 1, wobei die Informationen einen die Speicherfolie (13) eindeutig identifizierenden Identifikationscode darstellen.

3. System nach Anspruch 1, wobei die Röntgenaufnahmevorrichtung (12) dazu eingerichtet ist, die Aufnahmeparameter an die Schreib-/Lesevorrichtung (18) zu übertragen.

4. System nach einem der vorhergehenden Ansprüche, wobei der Datenträger (16) ein RFID-Transponder ist.

5. System nach einem der vorhergehenden Ansprüche, wobei die Speicherfolie (13) eine optisch lesbare Kennzeichnung aufweist und
die Datenvorrichtung dazu ausgelegt ist, die optisch lesbare Kennzeichnung mittels der Auslesevorrichtung (14) zu erfassen.

6. System nach Anspruch 5, wobei die optisch lesbare Kennzeichnung ein Strichcode oder ein QR-Code sind.

7. Verfahren zur Bereitstellung von Informationen für eine Auslesevorrichtung (14), mit den Schritten:
Durchführen (S1), mittels einer Röntgenaufnahmevorrichtung (12), eines Belichtungsvorgangs einer Speicherfolie (13);
Beschreiben (S2) eines Datenträgers (16), welcher der Speicherfolie (13) fest zugeordnet ist, mit Informationen, welche den Belichtungsvorgang charakterisieren, wobei es sich bei den Informationen um die Aufnahmeparameter bei der Röntgenaufnahme handelt und wobei die Aufnahmeparameter eine Spannung, eine Stromstärke, eine Belichtungszeit, eine Dosis, ein Dosisflächenprodukt und/oder einen Blendenwert umfassen;
Auslesen (S3) des Datenträgers (16); und
Auslesen (S4) der Speicherfolie (13) unter Berücksichtigung des Ausleseergebnisses des Datenträgers (16) und wobei das Auslesen der Speicherfolie (13) an die Aufnahmeparameter der Röntgenaufnahmevorrichtung angepasst wird.

## Claims

1. System (10) with
- a radiographic device (12) for recording a radiograph on an imaging plate (13) and
- a readout device (14) for the imaging plate (13), wherein the imaging plate (13) comprises a data carrier (16), wherein
the radiographic device (12) and/or the readout device (14) includes a data device (18, 20) with a write/read device for writing imaging parameters related to the radiograph to the data carrier (16) and for reading information stored on the data carrier (16), wherein the imaging parameters include a voltage, a current, an exposure time, a dose, a dose-area product and/or an aperture value, wherein the write/read device (20) is configured to transmit the information, which has been read, to the readout device (14), so that the imaging parameters used for the recording of the radiograph are available to the readout device (14) for a readout of the imaging plate (13) prior to the readout process, and wherein the readout of the imaging plate (13) can be adapted to the imaging parameters.

2. System according to Claim 1, wherein the information represents an identification code uniquely identifying the imaging plate (13).

3. System according to Claim 1, wherein the radiographic device (12) is configured to transmit the imaging parameters to the write/read device (18).

4. System according to one of the preceding claims, wherein the data carrier (16) is an RFID transponder.

5. System according to one of the preceding claims, wherein the imaging plate (13) comprises an optically readable identification and the data device is configured to detect the optically readable identification by means of the readout device (14).

6. System according to Claim 5, wherein the optically readable identification is a barcode or a QR code.

7. Method for providing information for a readout device (14), with the following steps:
performing (S1), by means of a radiographic device (12), an exposure process of an imaging plate (13);
writing (S2) information, which characterizes the exposure process, to a data carrier (16), which is permanently assigned to the imaging plate (13), wherein the information is the imaging parameters for the radiograph and wherein the imaging parameters include a voltage, a current, an exposure time, a dose, a dose-area product and/or an aperture value;
readout (S3) of the data carrier (16); and
readout (S4) of the imaging plate (13), taking into consideration the result of the readout of the data carrier (16) and wherein the readout of the imaging plate (13) is adapted to the imaging parameters of the radiographic device.

## Revendications

1. Système (10) comprenant
- un dispositif d'enregistrement radiographique (12) destiné à enregistrer une image radiographique sur une plaque d'imagerie (13), et
- un dispositif de lecture (14) pour la plaque d'imagerie (13), la plaque d'imagerie (13) présentant un support de données (16), dans lequel
le dispositif d'enregistrement radiographique (12) et/ou le dispositif de lecture (14) comprennent un dispositif de données (18, 20) doté d'un dispositif d'écriture/lecture destiné à inscrire des paramètres d'enregistrement concernant l'enregistrement radiographique sur le support de données (16) et à lire des informations mémorisées sur le support de données (16), dans lequel les paramètres d'enregistrement comprennent une tension, une intensité de courant, une durée d'exposition, une dose, un produit dose-surface et/ou une valeur de diaphragme, dans lequel
le dispositif d'écriture/lecture (20) est conçu pour transmettre les informations lues au dispositif de lecture (14) de sorte que les paramètres d'enregistrement utilisés lors de l'enregistrement de l'image radiographique sont disponibles pour le dispositif de lecture (14) pour une lecture de la plaque d'imagerie (13) avant le processus de lecture, et dans lequel
la lecture de la plaque d'imagerie (13) peut être adaptée aux paramètres d'enregistrement.

2. Système selon la revendication 1, dans lequel les informations représentent un code d'identification qui identifie univoquement la plaque d'imagerie (13).

3. Système selon la revendication 1, dans lequel le dispositif d'enregistrement radiographique (12) est conçu pour transmettre les paramètres d'enregistrement au dispositif d'écriture/lecture (18).

4. Système selon l'une quelconque des revendications précédentes, dans lequel le support de données (16) est un transpondeur RFID.

5. Système selon l'une quelconque des revendications précédentes, dans lequel la plaque d'imagerie (13) présente une identification lisible optiquement et
le dispositif de données est conçu pour détecter l'identification lisible optiquement au moyen du dispositif de lecture (14).

6. Système selon la revendication 5, dans lequel l'identification lisible optiquement est un code à barres ou un code QR.

7. Procédé de fourniture d'informations pour un dispositif de lecture (14), comprenant les étapes consistant à :
effectuer (S1) un processus d'exposition d'une plaque d'imagerie (13) au moyen d'un dispositif d'enregistrement radiographique (12),
inscrire (S2) sur un support de données (16), qui est associé de manière fixe à la plaque d'imagerie (13), d'informations qui caractérisent le processus d'exposition, dans lequel les informations sont les paramètres d'enregistrement lors de l'enregistrement radiographique, et dans lequel les paramètres d'enregistrement comprennent une tension, une intensité de courant, une durée d'exposition, une dose, un produit dose-surface et/ou une valeur de diaphragme ;
lire (S3) le support de données (16) ; et
lire (S4) la plaque d'imagerie (13) en tenant compte du résultat de lecture du support de données (16), et dans lequel la lecture de la plaque d'imagerie (13) est adaptée aux paramètres d'enregistrement du dispositif d'enregistrement radiographique.
